# EUROPEAN PATENT SPECIFICATION

(11) **EP 3 240 495 B1**
(45) Date of publication and mention of the grant of the patent: **11.09.2024**
(21) Application number: 16713101.0
(22) Date of filing: 22.03.2016
(51) Int. Cl.: A61B 34/10, A61F 9/008

(54) **SYSTEMS FOR THE OPTIMIZATION OF LASER PHOTOCOAGULATION**
SYSTEME ZUR OPTIMIERUNG DER LASERPHOTOKOAGULATION
SYSTÈMES POUR L'OPTIMISATION D'UNE PHOTOCOAGULATION AU LASER

(30) Priority: 23.03.2015 US 201562136935 P; 23.02.2016 US 201615050903
(43) Date of publication of application: 08.11.2017
(73) Proprietor: Alcon Inc., 1701 Fribourg (CH)
(72) Inventor: CLAUS, Michael John, Irvine, California 92618 (US); SANCHEZ, Robert, Oceanside, California 92057 (US); HEEREN, Tammo, Lake Forest, California 92630 (US); OLIVERA, Argelio Michael, Mission Viejo, California 92691 (US); PAPAC, Michael, Lake Forest, California 92630 (US); YU, Lingfeng, Lake Forest, California 92630 (US); REN, Hugang, Lake Forest, California 92630 (US)
(74) Representative: Murgitroyd & Company
(86) International application number: PCT/IB2016/051618
(87) International publication number: WO 2016/151491

(56) References cited:
- US-A1- 2013 116 670
- US-A1- 2014 257 257
- US-A1- 2014 307 078

## Description

### TECHNICAL FIELD

The present disclosure relates to systems for optimizing laser photocoagulation. Particularly, this disclose relates to systems for optimizing photocoagulation in ophthalmology.

### BACKGROUND

Over time, one or more locations of the retina of an eye may develop defects due to injury or disease. Laser photocoagulation may include the use of laser energy to precisely and finely cauterize one or more of the locations on the retina to provide therapeutic benefits. Some of these defects may be caused by various diseases or conditions. For example, diseases for which laser photocoagulation may be utilized include age related macular degeneration ("AMD"), diabetic retinopathy, retinal ischemia, arterial and venous occlusions, central serous chorioretinopathy, neovascularization of the choroid or retina, glaucoma, retinopathy of prematurity retinal tears or breaks, retinal detachment, lattice degeneration, posterior capsular opacification ("PCO"), and some ocular tumors.

Reference is made to the documents US 2013/0116670 A1, US 2014/0257257 A1, US 2014/0307078 Alwhich have been cited as representative of the state of the art.

### SUMMARY

It will be appreciated that the scope of the invention is defined by the claims.

According to one aspect, the disclosure describes a surgical optimization system that may include an imaging device adapted to receive imaging data of a tissue at a treatment location; and a treatment delivery device adapted to apply a treatment to the tissue at the treatment location; and a treatment control device. The imaging device and the treatment device may be coupled to the treatment control device. The treatment control may include a processor adapted to identify the presence of an abnormality of the tissue based on the received imaging data; determine a treatment plan using the identified abnormality; and deliver a treatment to the abnormality according to the treatment plan via the treatment delivery device.

A method to optimize treatment of a tissue is described for illustration, not being part of the claimed subject-matter. The method may include visualizing a tissue with an imaging device to obtain imaging data of the tissue; identifying, using an algorithm, an abnormality of the tissue based on the imaging data; generating, with a processor, a plan to treat the abnormality; and delivering treatment to the abnormality of the tissue according to the treatment plan.

The various aspects may include one or more of the following features. The processor may be adapted to identify a particular type of abnormality based on the received imaging data. The tissue at the treatment location may be a retinal tissue, and a type of identified abnormality may be one of a venous occlusion, a macular edema, a microvascular abnormality, a retinal break, a retinal tear, or an ocular tumor. The imaging device may be an OCT device. The processor may be adapted to determine treatment parameters for treating the identified abnormality. The treatment parameters may include one of a location to apply a treatment, a size of the location to be treated, locations excluded from treatment, and a laser power to be used for treatment.

Delivery of the treatment to the abnormality according to the treatment plan via the treatment delivery device may be performed autonomously by the treatment control device. Delivery of the treatment to the abnormality according to the treatment plan via the treatment delivery device may be performed upon receipt of a user input. The user input may include alignment of a target indicator with a treatment location of the abnormality. The processor may be adapted to update the treatment plan during the course of the treatment.

The various aspects may also include one or more of the following features. Delivering treatment to the abnormality of the tissue according to the treatment plan may include delivering treatment with a treatment delivery device. The treatment delivery device may include a treatment laser. Visualizing a tissue with an imaging device to obtain imaging data of the tissue may include visualizing the tissue with an OCT device. An algorithm used for identifying an abnormality of the tissue based on the imaging data may include an image processing algorithm. Generating, with a processor, a plan to treat the abnormality may include at least one of identifying a treatment location of the abnormality, a size of a treatment location of the abnormality, a power setting to be applied to an identified treatment location, and a location to be excluded from treatment. Delivering treatment to the abnormality of the tissue according to the treatment plan may include automatically delivering treatment to the abnormality according to the treatment plan. Delivering treatment to the abnormality of the tissue according to the treatment plan may include delivering treatment to the abnormality according to the treatment plan upon receipt of a user input. The treatment plan may be updated as the treatment is being delivered to the abnormality. The treatment plan may be registered with a real-time image of the tissue.

It is to be understood that both the foregoing general description and the following detailed description are exemplary and explanatory in nature and are intended to provide an understanding of the present disclosure without limiting the scope of the present disclosure. In that regard, additional aspects, features, and advantages of the present disclosure will be apparent to one skilled in the art from the following detailed description.

### BRIEF DESCRIPTION OF THE DRAWINGS

FIG. 1 is a schematic illustration of an example system for treating tissue abnormalities.
FIG. 2 is an example image that includes a tissue shown in a first view and imaging data of a portion of the tissue shown in a second view.
FIG. 3 is a flow diagram of an example algorithm for automatically detecting a retinal feature.
FIG. 4A is an example two-dimensional OCT image of a portion of a retina.
FIG. 4B is an example three-dimensional OCT image of a portion of a retina.
FIG. 5A is an example two-dimension OCT image of the portion of the retina shown in FIG. 4A with boundaries of an ILM layer and RPE layer identified.
FIG. 5B is an example three-dimensional OCT image of the portion of the retina shown in FIG. 4B with boundaries of an ILM layer and RPE layer identified.
FIG. 6 is an example chart that represents a thickness profile of the neurosensory retina.
FIG. 7A is an example two-dimensional OCT image that includes an indication of a detected retinal break.
FIG. 7B shows an indication surrounding a suspected retinal break combined with a fundus image of an eye.
FIG. 7C shows a pseudo color map that may be generated based on a detected retinal abnormality.
FIG. 8 is an example image showing a tissue having identified abnormalities and selected treatment locations associated therewith.
FIG. 9 shows the image of FIG. 8 with some of the selected treatment locations identified as having been treated.
FIGs. 10 and 11 illustrate example retinopexies applied to a retina.
FIG. 12 is an example method for optimizing tissue treatment.

### DETAILED DESCRIPTION

The present disclosure relates to medical treatment. Particularly, the present disclosure describes methods, apparatuses, and systems for optimizing laser photocoagulation in ophthalmology. In some instances, the laser photocoagulation may be fully automated, requiring only minor input from a user, such as a physician or other medical professional. In other instances, a user may have varying degrees of input during the laser photocoagulation. Additionally, in some instances, an apparatus embodying and/or used for the optimized laser photocoagulation may be a stand-alone device or system. In other instances, the apparatus may be incorporated into a surgical console that is operable to perform a plurality of surgical procedures.

The description is provided generally in the context of ophthalmology. However, ophthalmology is merely provided as an example field in which the presented subject matter may be used. Thus, the scope of the disclosure is not so limited. Rather, the subject matter described herein may be utilized in other applications, including applicable to other medical arts or even areas outside of the medical arts. Thus, the scope of the disclosure is not limited to ophthalmic applications. For example, the aspects of the disclosure may be applicable to other types of medical conditions and surgical procedures unrelated to ophthalmology. Further, the scope of the disclosure is not limited to laser photocoagulation treatments. Rather, other types of treatments, both within and outside of ophthalmology, are within the scope of the present disclosure.

Additionally, a retinal laser photocoagulation procedure is described. However, this, too, is provided only for illustrative purposes and is not intended to limit the scope of the disclosure. As explained above, the present disclosure may be applicable to both other types of ophthalmic surgical procedures as well as surgical procedures outside of the ophthalmology. Further, the present disclosure may be applicable outside of the medical arts.

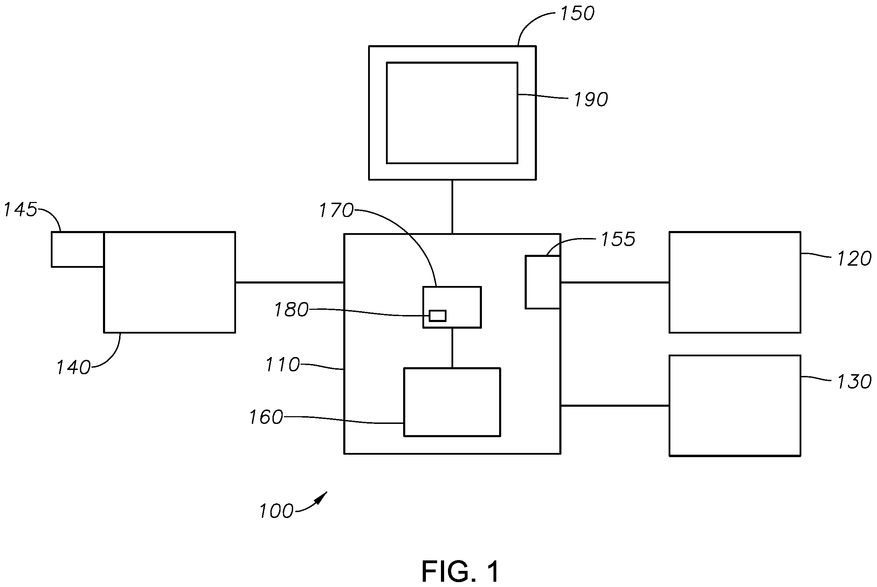

FIG. 1 shows an example system 100 that may be used to perform optimized laser photocoagulation procedures. The system 100 is operable to provide laser energy to perform laser photocoagulation of a portion of an eye, such as a retina. In some instances, the system 100 is operable to perform one or more of the following features: 1) visualize the ocular tissue, including obtaining imaging data of the ocular tissue; 2) identify abnormalities and their locations on an ocular tissue for treatment using the imaging data, including tracking the identified locations of the abnormalities; 3) determine appropriate laser parameters to appropriately perform a laser treatment of the abnormalities on the ocular tissue; and 4) treat the abnormalities, which may include, in some instances, firing a laser to treat the identified locations.

Further, in some implementations, the system 100 may also provide a "heads-up display" overlaid onto an image of an ocular tissue. The heads-up display may provide information to a user associated with a laser photocoagulation treatment. For example, the heads-up display may overlay one or more selected target locations for treatment onto the image of the ocular tissue. The selected target locations that have not yet been treated may be represented in one way (e.g., such as by way of represented symbol, color, character, etc.) and treated target locations in a manner different from the untreated target locations. The heads-up display may also provide a laser aiming indication. The laser aiming indication may identify a location on the ocular tissue where laser energy would be delivered if laser firing occurred. The laser aiming indication may be tracked real time and indicate to a user an instantaneous location where laser energy would impact the ocular tissue if the laser were fired.

The system 100 may include a laser control device 110, a laser delivery device 120, an imaging device 130, and a display 150. A microscope 140 may also be included. The laser control device 110 may include a treatment laser 155. The treatment laser 155 may be operably coupled to laser delivery device 120. In some implementations, the treatment laser 155 may be included with or otherwise form a part of the laser delivery device 120. In some implementations, the laser delivery device 120 may be operable to direct laser energy to a particular location on an ocular tissue. In some instances, the laser delivery device 120 may be a laser probe. The imaging device 130 may be operable to receive an image of an area of an ocular tissue. An image provided by the imaging device 130 may include imaging data, such as imaging data indicating tissue structures along a depth of the ocular tissue. The imaging device 130 may be utilized to image a portion of an ocular tissue for which imaging data is desired.

In some instances, the laser delivery device 120 and the imaging device 130 are or form parts of separate devices. For example, in some instances, the laser delivery device 120 may be a laser probe that is insertable, at least in part, into a portion of the eye. The imaging device 130 may be or form a portion of a separate device operable to receive and transmit an image of the ocular tissue and/or data representative thereof to laser control device 110 or other part of system 100. For example, the imaging device 130 may be an optical coherence tomography ("OCT") probe that is insertable, at least in part, into an eye. In other instances, the imaging device 130 may be operable to obtain infrared imaging data, retinal topography data, or any other type of data containing information usable to identify tissue abnormalities. One or more of the abnormalities may be determined to be suitable for laser photocoagulation treatment.

The transmitted image and/or image data from the imaging device 130 may be displayed to a user in any desired fashion. For example the received image and/or data may be displayed with a monitor, a microscope (e.g., within an eyepiece of a surgical microscope), as a data model representative of the ocular tissue, or in any other desired manner. In other instances, the laser delivery device 120 and the imaging device 130 may form or form part of a single device. Further, the system 100 may include a plurality of laser delivery devices 120 and/or imaging devices 130.

The microscope 140 may also be utilized to obtain an image of a portion of an eye. For example, the microscope 140 may be operable to obtain an image of an eye's retina or a portion thereof. The image of the retina may be observed directly by a user via the eyepiece 145. In some instances, the image obtained by the microscope 140 may be transmitted to a separate display, such as display 150. Thus, in some instances, the system 100 may include multiple components for observing a tissue for treatment. For example, the microscope 140 may be used to view a retina through the cornea and lens of the eye. The image data provided by the microscope 140 may encompass a large portion of the retina. In other instances, the image data may encompass a smaller portion of the retina. The imaging device 130 may also be able to obtain data that may be used in conjunction with the image data provided by the microscope 140. For example, the image data provided by the microscope 140 may include a visual image of the retina while the imaging device 130 may be operable to obtain OCT data of an area of the retina within the visual image. For example, the OCT data may include depth data along one or more scan lines of the tissue. Thus, the OCT data provides virtual cross-sectional information of the tissue taken along the one or more scan lines. In some instances, the imaging device 130 may form part of the microscope 140. In other implementations, the laser delivery device 120 may form part of the microscope 140. In still other implementations, both the imaging device 130 and the laser delivery device 120 may form part of the microscope 140.

While the imaging device 130 may be an OCT instrument inserted into the eye, the imaging device 130 may be a device operable to obtain OCT data prior to or instantaneously during a surgery without insertion into the eye. For example, in some instances, the imaging device 130 may be operable to obtain OCT data through the cornea and lens of the eye. Particularly, in some instances, the imaging device 130 may form part of the microscope 140. Further, the OCT data may be obtained through the cornea and lens of the eye.

In some instances, the system 100 may be a discrete, single purpose system. In other instances, the system 100 may be incorporated into a multifunctional system operable to perform laser photocoagulation as well as other surgical procedures. Thus, in some instances, the system 100 may be an integrated subsystem of a multifunctional surgical console.

The system 100 may include a processor 160 and a memory device 170 in communication with the processor 160. The memory device 170 may include any memory or module and may take the form of volatile or non-volatile memory including, without limitation, magnetic media, optical media, random access memory (RAM), read-only memory (ROM), removable media, or any other suitable local or remote memory component. Memory device 170 may contain, among other items, a laser control application 180. The laser control application 180 may provide instructions for operating aspects of the system 100. For example, laser control application 180 may include instructions for controlling the laser control device 110.

Memory 170 may also store classes, frameworks, applications, backup data, jobs, or other information that includes any parameters, variables, algorithms, instructions, rules, or references thereto. Memory 170 may also include other types of data, such as environment and/or application description data, application data for one or more applications, as well as data involving virtual private network (VPN) applications or services, firewall policies, a security or access log, print or other reporting files, HyperText Markup Language (HTML) files or templates, related or unrelated software applications or sub-systems, and others. Consequently, memory 170 may also be considered a repository of data, such as a local data repository from one or more applications, such as laser control application 180. Memory 170 may also include data that can be utilized by one or more applications, such as the laser control application 180.

Laser control application 180 may include a program or group of programs containing instructions operable to utilize received data, such as in one or more algorithms, to determine a result or output. The determined results may be used to affect an aspect of the system 100. The laser control application 180 may include instructions for controlling aspects of a treatment laser, such as treatment laser 155 for example. The application 180 may include instructions, such as one or more algorithms, for determining and controlling laser parameters. Control of the laser parameters may be premised on information inputted by a user and/or data received into the system, such as by one or more sensors. The one or more sensors may be included with or otherwise in communication with the system 100. For example, inputted information may be the imaging data received from the imaging device 130 and/or microscope 140. The laser control application 180 may determine one or more adjustments to the operation of the system 100. The adjustments may be implemented by one or more transmitted control signals to one or more components of system 100, such as, for example, the laser control device 110. While an example system 100 is shown, other implementations of the system 100 may include more, fewer, or different components than those shown.

In some instances, the laser control application 180 may provide instructions to obtain one or more images of an ocular tissue for treatment, identify one or more areas of the ocular tissue for laser photocoagulation treatment, generating a laser treatment plan, and delivering laser treatment to the one or more areas of the ocular tissue. The laser control application 180 may also include instructions for controlling one or more components of the system 100 and/or peripheral device coupled to the system 100. For example, in some implementations, the laser control application 180 may include instructions for controlling aspects of laser control device 110, the treatment laser 155, laser delivery device 120, imaging device 130, and/or display device 140. Further, the laser control application 180 may include instructions to generate a heads-up display for providing information to a user.

The processor 160 is operable to execute instructions and manipulate data to perform the operations of the system 100, e.g., computational and logic operations, and may be, for example, a central processing unit (CPU), a blade, an application specific integrated circuit (ASIC), or a field-programmable gate array (FPGA). Although FIG. 1 illustrates a single processor 160 in the laser control device 110, multiple processors 160 may be used according to particular needs and reference to processor 160 is meant to include multiple processors 160 where applicable. In some implementations, the processor 160 may include one or more microprocessors. The processor 160 may be adapted for receiving data from various components of the system 100 and/or devices coupled thereto, process the received data, and transmit data to one or more of the components of the system 100 and/or devices coupled thereto in response. In the illustrated example, processor 160 executes laser control application 180. The processor 160 may be operable to control various aspects of the system 100. For example, the processor 160 may form at least part of a controller operable to control firing of treatment laser 155 to perform a laser coagulation procedure. A variety of peripheral devices may also be coupled to the system 100, such as storage devices (hard disk drive, CD ROM drive, etc.), printers, and other input/output devices.

The display 150 displays information to a user, such as a medical practitioner. In some instances, the display 150 may be a monitor for visually displaying information. In some instances, the display 150 may operate both as a display and an input device. For example, the display 150 may be a touch sensitive display in which a touch by a user or other contact with the display produces an input to the system 100. In some instances, the display 150 may present information to the user via a graphical user inter face ("GUI") 190.

The display 150 may be utilized to display an image of a surgical site, such as an image of an ocular tissue. In some instances, the display 150 may be operable to display sensed data in the form of a model. For example, sensed data may be used to display a computer-generated model of a tissue or other portion of physical anatomy. The displayed model may be in the form of a three-dimensional model, two-dimensional model, or other type of model. A user, such as a medical practitioner, may utilized the display 150 as a source of information that includes image and other visual information. An eyepiece 145 of the microscope 140 may similarly be utilized to receive image and other information. In some instances, the eyepiece 145 may be operable to provide the same information as the display 150. In other instances, the information displayed by the eyepiece 145 may be different than that displayed by the display 150. The eyepiece 145 of the microscope 140 and the display 150 may be used simultaneously during a surgical procedure. In still other implementations, a heads-up display, described in more detail below, may also be displayed on the eyepiece 145 and/or the display 150. In other implementations, one of the eyepiece 145 or the display 150 may be eliminated.

GUI 190 may include a graphical user interface operable to allow the user, such as a medical practitioner, to interface with the system 100 for any suitable purpose, such as viewing application or other system information. For example, GUI 190 may provide information associated with a medical procedure, including detailed information related to a laser photocoagulation surgical procedure and/or operational aspects of the system 100.

Generally, GUI 190 may provide a particular user with an efficient and user-friendly presentation of information received by, provided by, or communicated within system 100. GUI 190 may include a plurality of customizable frames or views having interactive fields, pull-down lists, and buttons operated by the user. GUI 190 may also present a plurality of portals or dashboards. For example, GUI 190 may display an interface that allows users to input and define parameters associated with the laser control device 110, the treatment laser 155, laser delivery device 120, the imaging device 130, the microscope 140, display 150, or any other part of the system 100. It should be understood that the term graphical user interface may be used in the singular or in the plural to describe one or more graphical user interfaces and each of the displays of a particular graphical user interface. Indeed, reference to GUI 190 may indicate a reference to the front-end or a component of laser control application 180 without departing from the scope of this disclosure. Therefore, GUI 190 contemplates any graphical user interface. For example, in some instances, the GUI 190 may include a generic web browser for inputting data and efficiently present results to a user. In other instances, the GUI 190 may include a custom or customizable interface for displaying and/or interacting with the various features of the laser control application 180, for example. In other implementations, the GUI 190 may be utilized for displaying and/or interacting with any other part of the system 100.

In operation, a patient may be prepared for a laser photocoagulation procedure. The microscope 140 may be placed in position relative to the patient's eye in order to obtain an image of the retina. This retinal image may provide the user, such as the surgeon or other medical professional, with an image of a portion of the retina. The microscope 140 may obtain an image of the retina through the cornea and lens of the eye.

The imaging device 130 may be utilized to obtain visualization of the retinal tissue. In some instances, the imaging device 130 may be introduced into the patient's eye to obtain the imaging data. In other instances, the imaging device 130 may form part of the microscope 140 and obtain the imaging data through the cornea and lens of the eye. Visualization may include obtaining imaging data of at least a portion of the retinal tissue. The imaging data may be used to determine retinal abnormalities. This imaging data may be OCT data, infrared imaging data, retinal topography data, or any other type of data usable to obtain or determine the presence of retinal abnormalities. In some instances, the imaging device 130 may be used to obtain the imaging prior to the laser photocoagulation procedure. In some instances, the imaging device 130 may be used to obtain the imaging data during the laser photocoagulation procedure. In some implementations, the imaging device 130 may be used to obtain imaging data both prior to and during the laser photocoagulation procedure. For the purpose of this example, the imaging device 130 is described in the context of an OCT probe. However, this is done for illustrative purposes only, and, as explained, the imaging device 130 may be any device to obtain data that may be used detect abnormalities in a retina or other ocular tissue.

In some instances, the imaging device 130 may be adapted to sense the retinal imaging data while external to the eye. For example, in some instances, imaging device 130 may form part of microscope 140 and obtain OCT data through microscope 140 while external to the eye. In some implementations, at least a portion of the imaging device 130 may be inserted into the eye to obtain the retinal imaging data. The imaging device 130 may be used to obtain real-time imaging data. In other instances, the imaging data provided by the imaging device 130 may be obtained preoperatively. The imaging data may be collected in a digital format that can be subsequently analyzed. In some implementations, raw image data may be displayed on a video monitor or other presentation device. For example, the raw image data may be displayed on display 150 or in eyepiece 145. Further, the imaging data may be stored, such as in memory device 170 of example system 100.

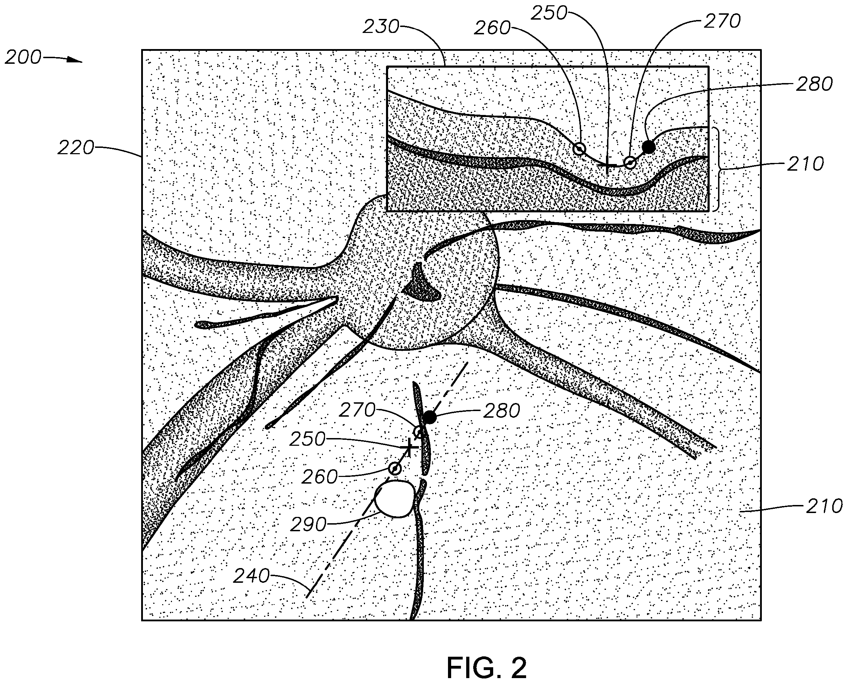

FIG. 2 shows an example image 200 of a patient's retina 210. The image 200 includes a primary view 220 and a detail view 230. In some implementations, the image 200 may be displayed on display 150 and/or eyepiece 145. In some implementations, the image 200 may form part of or otherwise be shown via GUI 190. The image of retina 210 may be obtained by the microscope 140.

The image 200 also includes a line 240 extending along a portion of the retina 210 in the primary view 220. The detail view 230 displays imaging data from the imaging device 130. In the present example, the detail view 230 shows OCT data (e.g., depth information) of the retina 210 along the line 240. The OCT data provides tomography data, which may include, for example, contour, shape, layer, and/or coloration information that may be used to detect retinal abnormalities. As indicated above, other types of sensors to detect or generate other types of data may be used to detect retinal abnormalities. In some implementations, abnormalities may be detected automatically by the system 100 according to one or more algorithms. The one or more algorithms may form part of the laser control application 180 or some other application.

The following description describes example algorithms for detecting a retinal abnormality. In some instances, abnormalities may be detected by obtaining OCT data of a location of a retina; segmenting the OCT data; generating a metric based on the segmented OCT data; and detecting a retinal abnormality based on the generated metric. Detection of a retinal abnormality may be indicated, for example, audibly, visually, tactilely, or a combination thereof. The OCT data may be in the form of OCT image data. Although retinal abnormalities are discussed in the context of algorithm 300, the scope is not so limited. Rather, the algorithms described herein may be utilized to detect other retinal features, such as, for example, retinal blood vessels.

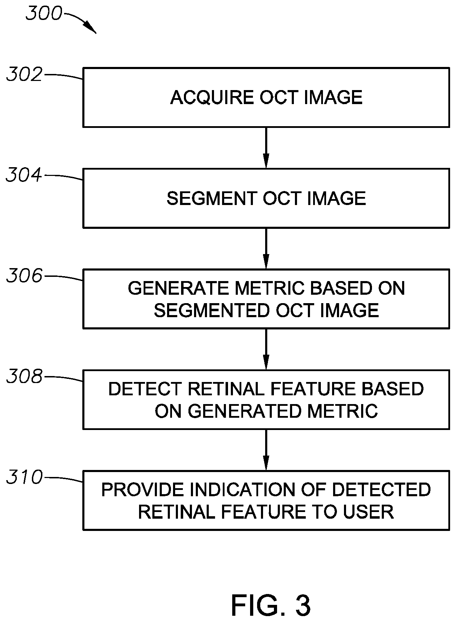

FIG. 3 provides a flow diagram of an example algorithm 300 to automatically detect a retinal feature using an ophthalmic system, such as system 100. Retinal abnormalities such as those described herein may be detected with the use of the algorithm 300. However, the scope is not so limited, and other retinal abnormalities other than those described may be detected using the algorithms described herein.

At step 302, the algorithm 300 may include acquiring an OCT image of a retina. At step 304, the algorithm 300 may include segmenting the OCT image. At step 306, the algorithm 300 may include generating a metric based on the segmented OCT image. At step 308, the algorithm 300 may include detecting a retinal abnormality based on the metric. At step 110, the algorithm 300 may include providing an indication of the detected retinal abnormality to a user (step 110). The steps of algorithm 300 may be performed by one or more components of an ophthalmic imaging system. For example, system 100 illustrated in FIG. 1 may be used. Further, algorithm 300 may be incorporated into an application stored on the imaging system 100. For example, all or a portion of the algorithm 300 may form part of the laser control application 180. In some instances, the algorithm 300 may form a part of one or more different applications, or the algorithm 300 may be in the form of a separate application.

The OCT system may be configured to split imaging light received from a light source into an imaging beam that is directed onto target biological tissue (e.g., by the imaging probe) and a reference beam that can be directed onto a reference mirror. The OCT system may be a Fourier domain (e.g., spectral domain, swept-source, etc.) or a time domain system. The OCT system may be further configured to receive the imaging light reflected from the target biological tissue (e.g., captured by the imaging probe, the external OCT system, etc.). The interference pattern between the reflected imaging light and the reference beam is utilized to generate images of the target biological tissue. Accordingly, the OCT system may include a detector configured to detect the interference pattern. The detector may include Charge-Coupled Detectors (CCDs), pixels, or an array of any other type of sensor(s) that generate an electric signal based on detected light. Further, the detector may include a two-dimensional sensor array and a detector camera.

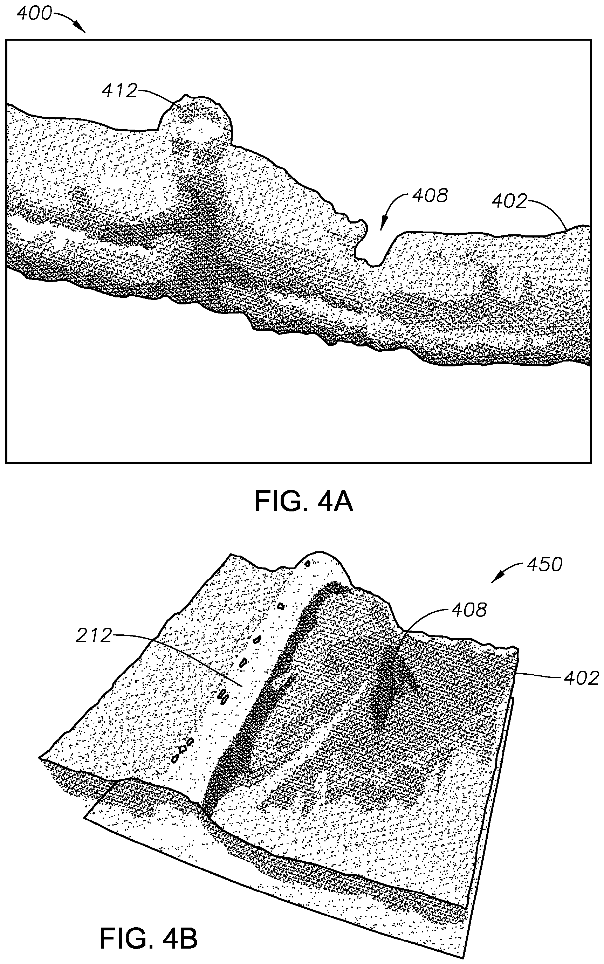

In some instances, the OCT data may be in the form of a two-dimensional OCT image. In some instances, the OCT data may be in the form of a three-dimensional OCT image. FIG. 4A shows a two-dimensional OCT image 400 of a portion of a retina 402, and FIG. 4B shows a three-dimensional OCT image 450 of a portion of the retina 402. A retinal break 408 is visible on the right side of FIGS. 4A and 4B. The retinal break 408, as well as other retinal abnormalities, may be automatically detected using the systems, methods, and devices described herein. A blood vessel 412 is visible on the left side of FIGS. 4A and 4B. Thus, other types of retinal features, such blood vessels and others, may be also be automatically detected using the systems, methods, and devices described herein.

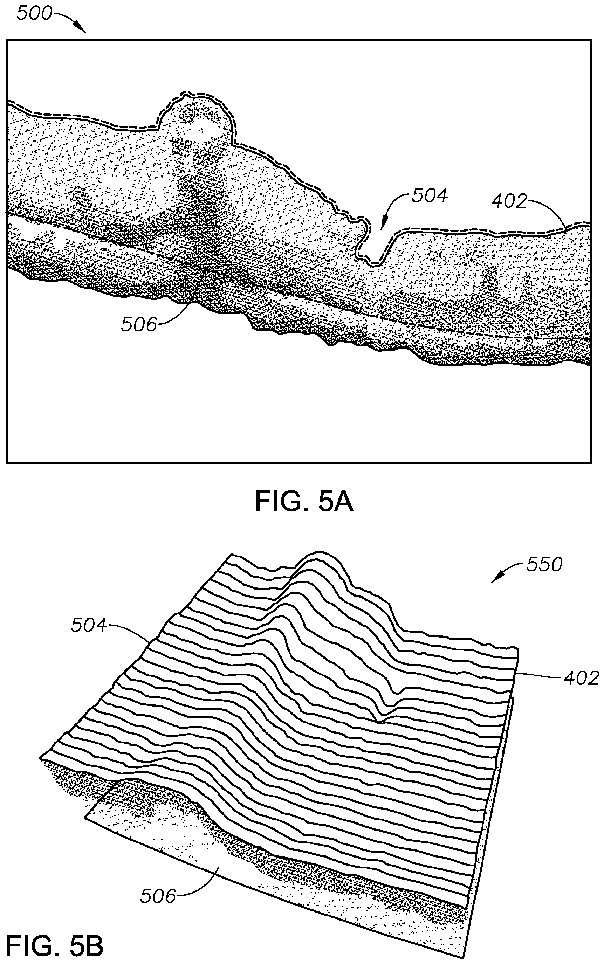

The OCT image may be segmented. Segmenting an OCT image includes identifying the different layers of the retina. For example, system 100 may identify one or more retinal layers using the data associated with the OCT image. Segmenting the OCT image may include identifying an inner limiting membrane (ILM), a nerve fiber layer, a ganglion cell layer, an inner plexiform layer, an inner nuclear layer, an outer plexiform layer, an outer nuclear layer, an external limiting membrane, a layer of rods and cones, a retinal pigment epithelium (RPE), and/or other retinal layer(s). FIG. 5A shows a two-dimensional OCT image 500 of the retina 402 with boundaries of an ILM layer 504 and an RPE layer 506 identified. Similarly, FIG. 5B provides the three-dimensional OCT image 550 of the retina 402 with boundaries of the ILM layer 504 and the RPE layer 506 identified.

One or more metrics associated with the retina may be generated based on a segmented OCT image. The metric may be a retinal layer parameter that objectively represents a geometry of one or more retinal layers using, for example, one or more numerical values. In some instances, the retinal layer parameter may be a thickness, an intensity, an intensity gradient, a phase, a speckle size, a vascular density, a blood flow velocity, an oxygenation, an elasticity, a birefringence property, a size, a volume, a concavity/convexity, and/or a radius of curvature of one or more retinal layers. For example, generating the metric may include determining a numerical representation of the concavity/convexity of the ILM. For example, a radius of curvature of the ILM in the area of the retinal abnormality may be determined. The retinal layer parameter may be determined using any number of retinal layers. For example, the retinal layer parameter may be determined using any one, two, three, four, or more retinal layers. Generating the metric may include determining a thickness of the neurosensory retina using, for example, the ILM and RPE. For example, the thickness of the neurosensory retina may include a distance between the ILM and RPE. A numerical representation of the thickness may be used as the metric. In some instances, the retinal layer parameter may be determined using one retinal layer and a strip of predefined thickness that surrounds the one retinal layer. One, two, or more metrics may be generated and utilized to evaluate the retina.

Detecting one or more retinal abnormalities may be based on the generated metric. The detected retinal abnormality may be a structural aspect of the retina that is indicative of a defect. For example, the retinal abnormality may be a break, a hole, a tear, a dialysis, a growth, a protrusion, a depression, a region with subretinal fluid, etc. Multiple retinal abnormalities and, in some instances, the types thereof, may be detected. The retinal abnormality or abnormalities may be detected using one or more of the metrics. For example, the thickness of the neurosensory retina and the concavity/convexity of the ILM may be utilized. Utilizing more than one metric may advantageously increase the certainty of retinal abnormality detection.

Detecting the retinal abnormality may include comparing the retinal layer parameter to a threshold. For example, when the generated metric includes a thickness of the neurosensory retina, detecting the retinal abnormality may include comparing the thickness to a threshold thickness. In some instances, a retinal abnormality may be detected when a retinal layer parameter, such as thickness of the neurosensory retina, among others, is greater than or less than a threshold value. For example, a retinal break or a retinal hole may be detected when a thickness is less than a threshold value. On the other hand, a growth or a protrusion of the retina may be detected when a thickness is greater than a threshold value. A threshold thickness may be in the range of, for example, 50 microns to 300 microns; 75 microns to 300 microns; 100 microns to 250 microns; or other suitable range. Generally, thickness varies along the retina. For example, the retina may vary in thickness at or near the fovea, peripheral retina, or other locations. As a result, a threshold value may be selected based on a position along the retina where the retinal abnormality is located.

Detecting the retinal abnormality using the generated metric may include determining whether the one or more retinal layers, such as the ILM, among others, has a concave or convex shape and/or the degree of the concavity or convexity (e.g., the radius of curvature). For example, an ILM in the area of a retinal abnormality that is concave may be indicative of a retinal break or a retinal hole, whereas an ILM that is convex may be indicative of a growth or a protrusion in the retina. Thus, detecting a retinal abnormality may include comparing a radius of curvature of the ILM in the area of the retinal abnormality to a threshold radius of curvature indicative of the presence of the retinal abnormality. A retinal abnormality may be detected when the radius of curvature is greater than or less than a threshold value. For example, a retinal break or a retinal hole may be detected when a concave portion of the ILM has a radius of curvature less than a threshold value. The threshold radius of curvature for detecting a retinal break may be in the range of, for example, between about 0.1 mm and about 12 mm; between about 1.0 mm and about 6 mm; or between about 2.0 mm and about 4.0 mm; including values such as 10 mm, 9 mm, 8 mm, 7 mm, 6 mm, 5 mm, 4 mm, 3 mm, 2 mm, 1 mm, or other suitable value. A combination of the concavity or convexity and the corresponding radius of curvature may be utilized to detect the retinal abnormality.

A threshold or thresholds used in detecting a retinal abnormality may be adaptive or patient-specific. For example, a threshold value may be a percentage difference in the neurosensory retina thickness compared to adjacent areas. Thus, a retinal abnormality may be detected when an area of the patient's neurosensory retina has a thickness greater than or less than, e.g., 50% of the thickness of adjacent areas. Similarly, a retinal abnormality may be detected when the radius of curvature of the ILM is greater than or less than, e.g., 50% of the radius of curvature of adjacent areas. The threshold can be between, for example, 1%-100%, 1%-75%, 1%-50%, 1%-25%, etc. Although a thickness of neurosensory retina and the radius of curvature of the ILM are discussed, these are used merely as examples. Thus, the scope of the disclosure is not so limited. Other metrics, such as thicknesses or radius of curvature of other layers of the retina or other retinal characteristics, such as one or more of those described above or others, may be used to locate and identify retinal abnormalities.

The one or more thresholds may be selected based on empirical data. For example, a collection or database of patient retinal data may be used to determine normalized or baseline retinal data. This baseline data may be used to obtain threshold values to detect retinal abnormalities. For example, a database containing thickness measurements of the neurosensory retina of patients with similar characteristics may be used to determine a normal range of thicknesses of the neurosensory retina. This normal range of thicknesses may be used to generate threshold thickness values for the neurosensory retina. Thus, a retinal abnormality may be detected when an area of the patient's neurosensory retina has a thickness outside of (e.g., greater than or less than) the normal range expected for the patient. In some instances, such empirical data may be used to determine a default threshold value, which may be adjusted based on patient specific characteristics. While this discussion specifically mentions thickness of the neurosensory retina, it is understood that other characteristics, such as the concavity, or convexity, or radius of curvature, and/or other metrics, can be similarly patient-specific or more generally applicable.

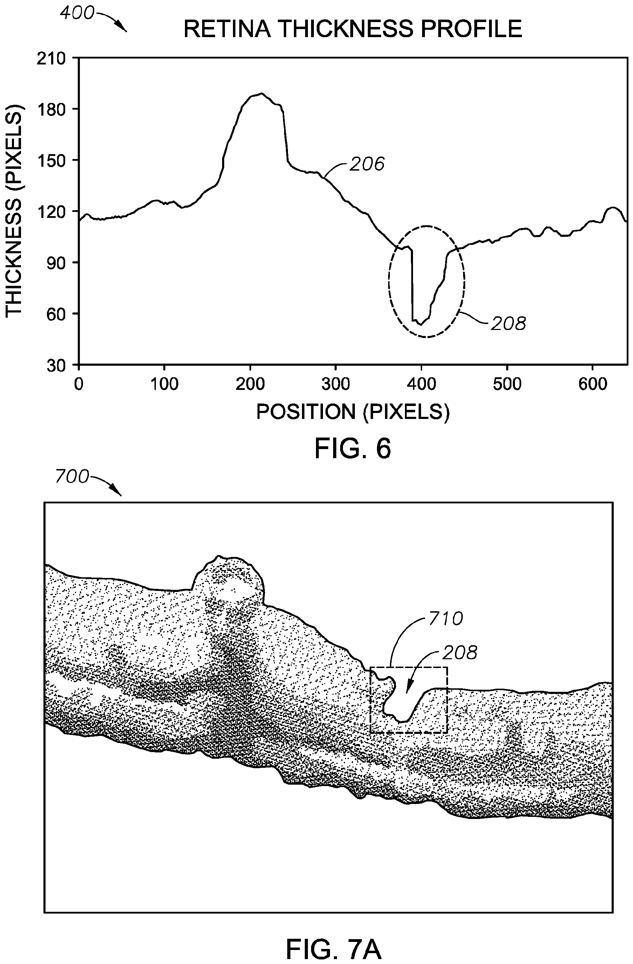

FIG. 6 shows a chart 600 that is representative of a thickness profile of the neurosensory retina. The data associated with the chart 400 may be based on the segmented OCT image. The x-axis of the chart 400 represents the position along the neurosensory retina in units of pixels. The y-axis represents the thickness of the neurosensory retina in units of pixels. A curve 206 represents the distance of the ILM from the RPE along the retina. The neurosensory retina thickness depicted in chart 400 may be the metric used to detect the retinal break 208. The retinal break 208 may be an area along the neurosensory retina with a thickness that is significantly different from adjacent areas (e.g., less than 50%) and/or an area with a thickness less than a fixed, normal range. While this discussion specifically mentions thickness of the neurosensory retina, it is understood that the concavity/convexity, radius of curvature, and/or other metrics can be similarly used to detect the retinal break or other retinal abnormality.

An indication of a detected retinal abnormality may be provided to a user. An audio, visual, and/or tactile indication may be provided using one or more devices to provide an audio, visual, and/or tactile indication. For example, display 150 shown in FIG. 1 may be utilized to provide a visual indication of the detected retinal abnormality. The indication may be used to alert a user, such as a surgeon, of the presence and/or position of the detected retinal abnormality. Particularly, the indication may be utilized to alert a user of the detected retinal abnormality during the course of a surgical procedure. As shown in FIG. 7A, a two-dimensional OCT image 700 includes an indication 710 of the detected retinal break 208. A visual indication, such as indication 710, may be in the form of a geometrical object positioned in relation to the detected retinal abnormality. For example, in some instances, the indication may be a geometrical representation in the form of a square, a circle, a polygon, an ellipse, or any other geometric shape positioned around retinal break 408. In some instances, the indication 710 may be overlaid on and/or otherwise combined with an OCT image, such as the OCT image 200 shown in FIG. 4A, and the combined OCT image may be output to an output device, such as the display 150. In other instances, other types of indications may be used either alone or in combination with each other.

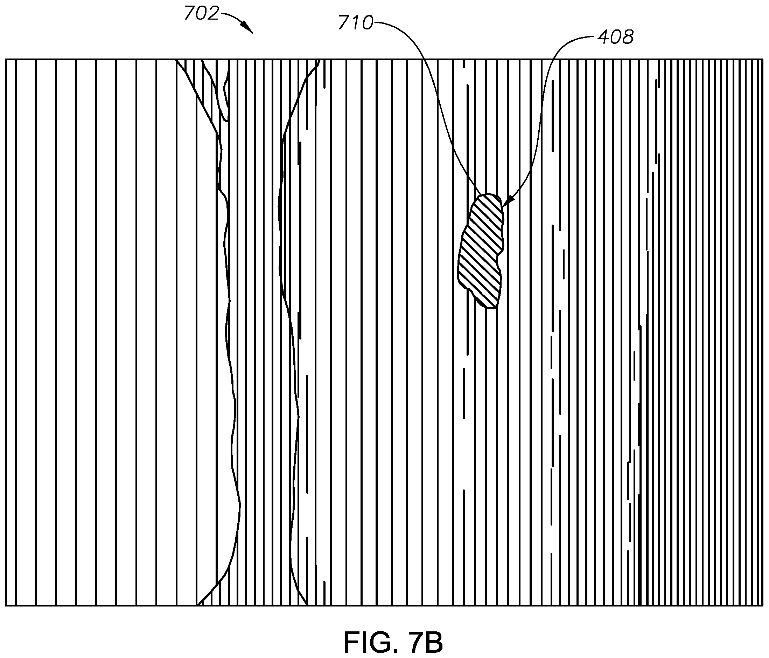

In some instances, an indication may have a shape that is based on a shape of the detected retinal abnormalities. For example, as shown in FIG. 7A, the indication 710 may surrounds the detected retinal break 208. In some instances, an indication may have a shape that conforms or corresponds to the detected retinal abnormality. For example, as shown in FIG. 7B, the indication 710 may be overlaid on and/or otherwise combined with a fundus image of the eye, such as fundus image 702. The combined fundus image may be output to the display 150. Again, other types of output, such as audible and/or tactile, may be provided to a user to indicate a retinal abnormality.

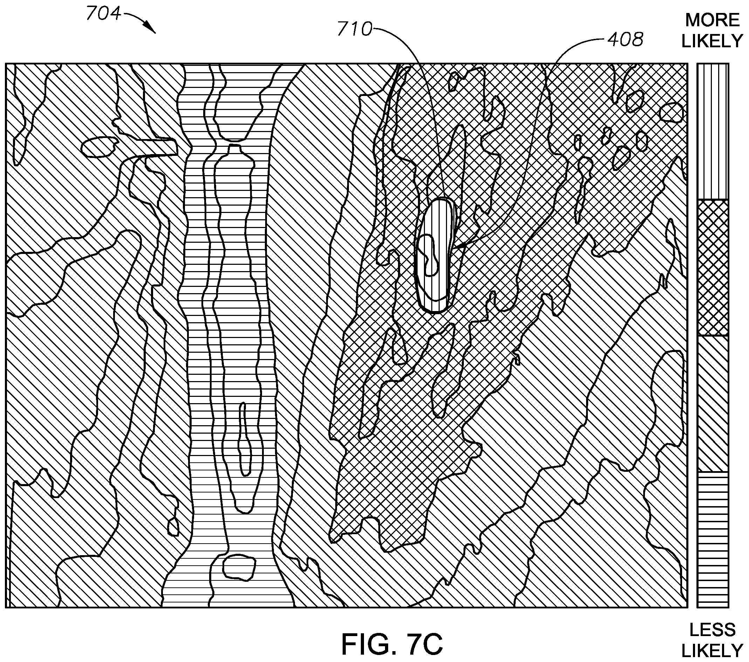

FIG. 7C shows a pseudo color map 704 that may be generated based on a detected retinal abnormality. In the example illustrated, the pseudo color map 704 is generated based on retinal break 408. The pseudo color map 704 may be representative of the likelihood of the presence of a retinal abnormality at a given location of the retina. The indication 710 may represent an area of the pseudo color map 704 where a retinal abnormality is likely present. The pseudo color map 704 may be output to the display 150, for example.

In some implementations, an indication, such as indication 710, may include other information. For example, an indication may include text, one or more other shapes or symbols, and/or other visual alerts. An indication may be variously positioned relative to the retinal abnormality. An indication may include an audible signal to alert the user/surgeon of the presence and/or position of a detected retinal abnormality. An indication may include tactile and/or haptic feedback to the surgeon.

Referring again to FIG. 2, the image 200 also includes a cursor 250 disposed at a location along the line 240. Another representation of the cursor 250 is also shown at a location along the retina 210 in the detail view 230. The location of the cursor 250 in the primary view 220 is linked to the location of the cursor 250 in the detail view 230, such that the location of the cursor 250 in the primary view 220 corresponds to the same location along the retina 210 shown in the detail view 230. That is, the location of the retina 210 indicated by the cursor 250 in the primary view 220 is the identical location on the retina 210 as identified by the cursor 250 in the detail view 230. Thus, a change in location of cursor 250 in the primary view 220 is reflected as a change in the location of the cursor 250 in the detail view 230 and vice versa.

A user may change location of the cursor 250 with the use of an input device. For example, a user may use a stylus or other digital input device. The locations selected with the use of cursor 250 may be identified by interaction of an input device with the presented imaging data. For example, a location may be identified by touching a stylus to display 150. The location touched by the stylus may be identified as a selected treatment location. Other input devices may be used to select a treatment location. For example, a mouse, keyboard, or touchscreen may be used. The selected treatment locations may then be registered with the particular location on the retina and stored digitally, for example, to maintain a correct registration between the position of a patient's eye and the location(s) of selected treatment locations(s). While treatment locations may be selected manually in some implementations, in other implementations, one or more selected treatment locations may be determined automatically by the system 100.

In some implementations, registration of the selected treatment locations and the real-time image of the retina 210 may be obtained with a retinal tracking device, in which retinal tracking facilitates providing to the user the treatment locations at all times regardless of patient eye orientation due to movement or microscope adjustment.

In some implementations, a retinal tracking device may include a fundus imager and a registration and tracking calculator. Example fundus imagers may include optical cameras, a line scan ophthalmoscopes operable to obtain line scan images, and confocal scanning ophthalmoscopes. Other imaging technologies may also be used to obtain retinal images. The fundus imager may be or form part of the imaging device 130, for example. Alternately, in some instances, the fundus imager may be an imaging separate from the imaging device 130.

The fundus imager may acquire live, i.e., real-time, retinal images. The registration and tracking calculator may receive and compare the live retinal images with a previously-obtained retinal image. For example, a preoperatively obtained retinal image may be used as the previously-obtained retinal image. Differences between the compared images may be detected by the registration and tracking calculator. These differences may indicate movement of an eye that has occurred in the time between when the two images were obtained. The registration and tracking calculator may then adjust the positions of the representations of the selected treatment locations so that the selected treatment locations remain accurately positioned relative to the appropriate locations on the retina on the image of the retina 210. Thus, the selected treatment locations remain registered with the actual locations on the retina selected for treatment.

Example retinal tracking devices may be similar to retinal tracking systems described in "A new real-time retinal tracking system for image-guided laser treatment", IEEE Trans Biomed Eng. 2002; 49(9): 1059-67. Such retinal tracking system includes a fundus imager and a tracking and registration calculator. The fundus imager acquires a real-time image of the retina and transfers the data to the tracking and registration calculator. The tracking and registration calculator receives the live retinal image from the fundus imager, processes the received retinal image, compares the processed retinal image with a processed retinal image that was previously obtained, determines whether the retina has moved during the time the two retinal images were taken by calculating a difference in position of one or more features on the retina between the two images to determine motion information of the retina, and adjusts the positions of the selected treatment locations so that the treatment locations remain accurately associated with their corresponding locations on the retina. Consequently, the selected treatment locations remain properly located on the retinal image notwithstanding any relative movement between the retina 210 and the treatment system 100.

As indicated above, the fundus imager may capture live images of the retina. In some implementations, the fundus imager may capture real-time images of the retina and operate the tracking and registration calculator continually to maintain registration of the selected treatment locations on a real-time basis, thereby compensating for eye movements that may be occurring continually.

The retinal images obtained by the fundus imager, as explained above, may be real-time images. Processing of the real-time retinal images may involve enhancing one or more aspects of the images' data, for example, to identify one or more parameters associated with the retina. The one or more parameters are then used to detect a feature and/or characteristic of the retina.

Processing the real-time retinal images may include image filtering. Image filtering may be utilized to remove noise contained within the image data. In some instances, image filtering may be accomplished by applying a moving window across an image to reduce noise. Processing may also include characterizing the retina. In some instances, processing of the retinal images may be used to identify vasculature characteristics of the retina. For example, processing may also include vessel segmentation, which extracts and identifies blood vessels in the retinal image. The vessels may be segmented based on edges between vessels and a non-vascular region of the retina. Processing may include vessel branch and crossover identification may include identifying where branches of vessels within the retina approach or cross over one another in the same region of retina. Other parameters detected may include vessel shapes; the shape, position, or center of optical nerve head; and fovea position. Other retinal features may also be used.

The identified parameters, such as vessel branches and crossovers, are then compared with those of a previously-obtained retinal image in order to locate and match identical features within the different retinal images. Based on this comparison, the tracking and registration calculator calculates a transformation matrix, which is a mathematical representation of the movement made by the retina in the time transpiring between the acquisitions of the different retinal images. Thus, this transformation matrix mathematically represents the difference in the retina's position between the two retinal images. The tracking and registration calculated applies the transformation matrix to adjust the selected treatment locations on a real-time retinal image that may be displayed in the eyepiece of a microscope and/or another display.

While FIG. 2 shows a single line 240, the image 200 may include multiple lines. Further, the lines may be designated by a user or according to an algorithm in any desired orientation. For example, in some instances, the lines defining OCT data may be defined real-time during the ophthalmic surgical procedure. As mentioned above, the OCT data may be obtained during the surgical procedure with the imaging probe 130. As explained above, the imaging probe 130 may form part of a microscope, such microscope 140, or may be or form part of a separate device. In other instances, the OCT data may be determined preoperatively. Preoperatively obtained OCT data may be registered with the real-time image 200 obtained during the surgical procedures. Thus, the preoperatively obtained OCT data is accurately located so that it is aligned with the portion of the retina that the OCT data represents. In still other implementations, both real-time OCT and preoperatively obtained OCT may be used together.

Referring again to the OCT data shown in the detail view 230 taken along line 240, the cursor 250 may be moved to any point along the line 240, such as by a user, for example. A user may select one or more locations along the line 240 to which a laser photocoagulation treatment may be applied. For example, FIG. 2 shows example selected treatment locations 260, 270, and 280. Although three selected treatment locations are shown, additional or fewer selected treatment locations may be present and/or selected. The selected treatment locations may be stored in memory (such as memory 170 shown in FIG. 1). Further, the positions of the selected treatment locations may be registered with the retinal image, such as with the use of an eye tracking device. Therefore, in some instances, once a user, such as a surgeon or other medical professional, has selected one or more locations on the retina for treatment, those selected locations are registered and remain associated with the selected location regardless of patient orientation, eye movement, microscope position, zoom or focus settings, or changes thereto.

In other implementations, the selected treatment locations may be selected automatically by the system 100. For example, in some implementations, laser control application 180 or another active application may include an algorithm that is operable to detect suspected retinal abnormalities without input from a user. For example, retinal abnormalities may be identified automatically with the use of one or more images of the retina. While the example algorithms discussed above utilize OCT image data to identify retinal abnormalities, other types of image data may be used to detect retinal abnormalities. For example, microvascular abnormalities of the retina may be automatically detected based on angiogram images, such as fluorescein angiogram, or OCT angiogram images. In some instances, a microvascular pattern and density can be quantified based on fractal analysis. A fractal analysis is a mathematical process that determines data densely of an image. Fractal analysis is used to analyze a fractal dimension or other fractal characteristics of a data set. By performing fractal analysis of, for example, the segmented vessels contained in an image, vasculature density information can be obtained. The presence of the vessels may be determined in a manner discussed above.

Fractal analysis may be achieved by using a box counting method. In box counting, a retinal image is overlaid with a series of square boxes of decreasing size. The number of boxes containing at least one pixel of retinal vessels is counted. A least squares regression slope between number of boxes and size of boxes yields fractal dimension, which represents the vessel density of the retina. Vascular density of a particular value may be representative of a particular type of abnormality. Further, different abnormalities may be representative by different vascular density values. The system 100 may automatically identify a retinal abnormality based on a detected vascular density. For example, in some instances, an abnormality may be determined using a look up table. An application running on the system 100, such as the laser control application 180, may contain a look up table that may include one or more abnormality type and its corresponding vascular density value. When a vascular density is determined from a retinal image, the system may automatically predict a retinal abnormality associated therewith. In some instances, the system 100 may automatically treat the predicted abnormality by identifying treatment locations, as explained in more details below, and applying treatment energy thereto. IN other instances, the system 100 may present the predicted abnormality to a user and await further user input.

A fractal analysis may result in a regional fractal dimension that represents a vascular density or pattern of that region. As explained, the regional fractal dimension may be used as a parameter for detecting vascular abnormalities. Other techniques may be used to detect retinal abnormalities. For example, in some instances, for example, vascular oxygen saturation, fluorescein angiogram data, and 3D OCT image data may be quantified to identify locations of the retina with abnormal function.

A microvascular abnormality may be detected using pre-operatively acquired fluorescein angiogram ("FA") images. The pre-operatively acquired FA images may be registered with a real-time retinal image and overlaid onto a real-time retinal image. The real-time retinal image with registered pre-operatively acquired FA images may be displayed on a display, such as display 150, or a microscope view presented within the eyepiece of a microscope, such as the eyepiece 145 of microscope 140. The pre-operatively acquired FA image registered onto a real-time retinal image may be described as an overlaid real-time image. An area of neovascularization may be presented as a bright area in the overlaid real-time retinal image. An adaptive threshold of the fundus FA signal can be used to identify areas of neovascularization.

Thresholding is a convenient way to segment objects contained in an image from a background also contained in the image. If that background is relatively uniform, a global threshold value can be used to binarize the image by pixel-intensity. Thus, a global threshold value is a single threshold value that is applied across an entire image to identify the object in the image apart from the background. An adaptive threshold is one in which a threshold value applied to an image varies. If a large variation in the background intensity of an image exists, adaptive thresholding (also known as local or dynamic thresholding) may produce better results. Adaptive thresholding calculates thresholds in a region of the image surrounding each pixel or group of pixels. These regions may be referred to as "local neighborhoods." The threshold value applied to a particular pixel is a weighted mean of the local neighborhood minus an offset value, and may be referred to as the adaptive threshold value. Generally, the offset value is a preset numerical value. The offset value adds flexibility to adjust and fine tune the ultimate threshold for better segmentation results. A value associated with the pixel may be compared to the adaptive threshold value to determine useful information about characteristics of the retina, such as to identify an object in the retinal image.

Another characteristic of the retina that may be identified using the techniques described herein is capillary nonperfusion. An area of capillary nonperfusion may be presented as an area that is darker or even blackened area relative to the surrounding tissue within the image. A threshold value of area mean signal strength can be used to identify capillary nonperfusion areas. That is, a threshold value of mean signal strength may be utilized to determine whether a measured mean signal strength is indicative of the presence of a capillary nonperfusion area. In some instances, this threshold may be an adaptive threshold. In some instances, the threshold may be a global threshold. An abnormality map for a blood vessel can then be developed based on the identified neovascularization or capillary nonperfusion area or areas.

In another implementation, a microvascular abnormality detection algorithm may be based on 3D OCT images. The whole 3D retinal vasculature network can be reconstructed based on 3D OCT information. The microvascular pattern and density are then quantified based on fractal analysis which generates a regional fractal dimension that characterizes the vascular density and vascular abnormality of that region.

Example abnormalities may include venous occlusions, macular edema, microvascular abnormalities, retinal breaks and tears, ocular tumors, as well as others. Thus, the system 100 may be operable to identify suspected retinal abnormalities and select one or more selected treatment locations in relation to the suspected retinal abnormality. The treatment locations automatically identified by the system, such as system 100, may be presented to a user, such as a surgeon, for review and/or modification prior to further development of further treatment options.

Selected treatment locations automatically identified by a laser photocoagulation system, such as system 100, may be dependent upon one or more factors or inputs. For example, the system may input received retinal information into a treatment planning algorithm. The treatment planning algorithm may return a pathological case or abnormality suggested by the received retinal data. Based on the identified pathological case or abnormality, the treatment planning algorithm proposes a treatment plan, such as by selecting one or more treatment locations to receive photocoagulation treatment energy. For example, in an instance where a retinal break or tear is identified by the treatment planning algorithm, one or more treatment locations may be registered with and indicated on a retinal image, such as a real-time retinal image. In the case of a retinal break or tear, the selected treatment locations may be placed so as to surround the break or tear. In the case of a microaneurysm, one or more treatment locations automatically selected by the treatment planning algorithm may be located directly on the location of the retina where the microaneurysm has been identified.

In some instances, the system will prompt the user to verify that the automatically selected treatment locations are acceptable before treatment is applied. In other instances, the treatment may be applied automatically upon determination of the selected treatment locations by the treatment planning algorithm without input from the user.

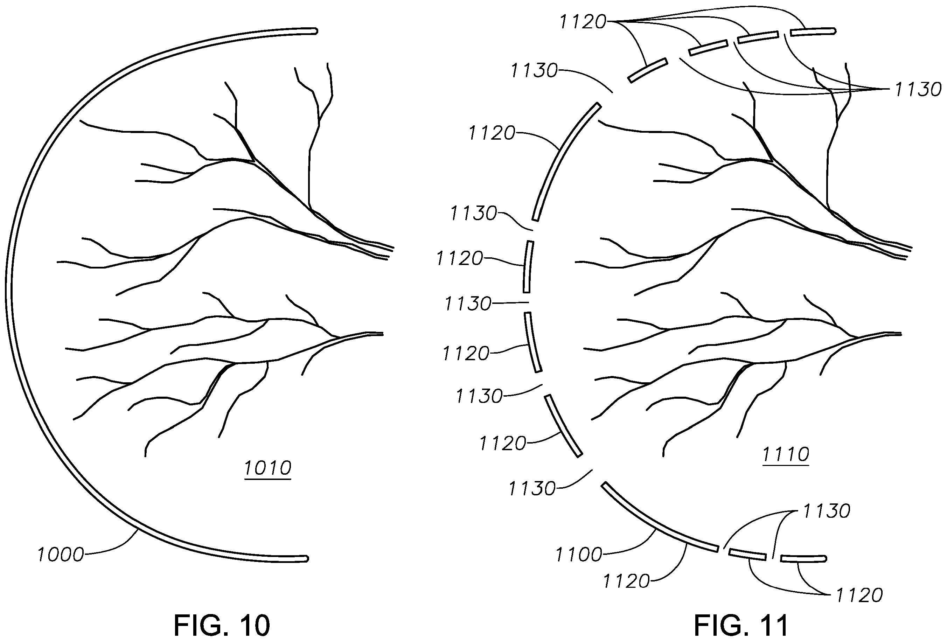

Treatment locations may also be selected to perform other types of procedures. For example, one or more treatment locations may be selected to perform a retinopexy. A retinopexy procedure includes applying laser energy to a location on a retina to create a burn the bonds the retina to the back of the eye. Retinopexies take on various forms. For example, a retinopexy may involve continuously applying laser energy (such as by continuously firing a laser) along a selected path on a retina. FIGs. 10 and 11 show a portion of a retina having retinopexy procedure performed thereon.

A selected path of the retinopexy may have a desired length. Also, the path may be, at least in part, arcuate, straight, or have any desired shape. As shown in FIGs. 10 and 11, the paths 1000 and 1100 along retinas 1010 and 1110, respectively, have generally curved shapes. In other instances, a retinopexy may be defined along a desired path but the laser may be fired along only one or more parts of the path. For example, for a desired path, a laser burn may be formed over a selected length, another length of the path may be unaffected (i.e., no laser energy is applied thereto), and another portion of the path may have laser energy applied thereto also to form a retinal burn. The path 1100 shown in FIG. 11 illustrates this type of treatment. The path 1100 includes a plurality of laser burns 1120 that are separated by untreated portions or gaps 1130. Thus, for any selected path, one or more portions of the path may be selected to have a retinal burn formed thereon for a desired length and an untreated portion (i.e., a portion that is not treated with laser energy and is, thus, unburned) may have any desired length. Still further, for a selected path, each portion that is designated to receive a retinal burn may be selected to have any desired length independent of any other portion designated to receive a retinal burn. Thus, in some instances, one or more portions of the path to be treated may have different lengths. In other instances, one or more of the potions of the path to be treated may have the same length. Similarly, the portions of the path that are to remain untreated (e.g., those portions of the path disposed between those portions of the path to be treated) may have any desired length. Thus, in some instances, one or more untreated portions of the path may have different lengths. In other instances, one or more untreated portions of the path may have the same length.

One particular, non-limiting example retinopexy that is within the scope of the disclosure is a 360° prophylactic retinopexy. A 360° prophylactic retinopexy includes the formation of a 360° retinal burn around an entire perimeter of a retina or a portion thereof. A 360° prophylactic retinopexy may be performed as a preventative measure. In some instances, a 360° prophylactic retinopexy may be performed during another ophthalmic surgical procedure in order to bond the retina to the back of the eye before a problem with the retina exists in cases where a medical professional, such an ophthalmologist, believes a retinal problem may occur or is likely to occur. This type of preventative measure may be performed during an ophthalmic surgical procedure in order to avoid the need to re-enter the eye at a later time. The selected path along which a 360° prophylactic retinopexy is performed may be continuous or may have one or more treated lengths separated by one or more untreated lengths, as explained above. Further, a prophylactic retinopexy need not be formed along a 360° path. Rather, the path may be less than 360° or greater than 360°. Still further, the start point of the path need not be the same as the end point of the path.

Identification of the path for a retinopexy and/or the location(s) to be treated along the path may be determined in the different ways described herein. Further, application of the laser energy to the path may also be applied in the different ways disclosed herein.

An algorithm operable to detect one or more locations on a retina for treatment may also be operable to determine one or more laser parameters used by the laser to treat the detected retinal abnormality. For example, the algorithm may be operable to determine laser power to be applied to each of the selected treatment locations, a duration of time laser energy is applied to one or more of the selected treatment locations, the size of the selected treatment locations to be treated, locations to exclude from treatment, as well as others parameters.

The parameters selected, such as the number and size of the selected treatment locations, laser power, as well as any other laser parameter, may be automatically determined based on, for example, the type of detected retinal abnormality, the size of the abnormality, the severity of the abnormality, and/or any other criteria. Selection of the treatment locations and the other laser parameters associated with treatment of a retinal abnormality, whether determined automatically by an algorithm or manually by a user, defines, at least in part, a treatment plan. The algorithm may optimize the treatment plan, for example, by selecting laser parameters to improve procedure effectiveness, reduce procedure timing, minimize cellular necrosis and vision loss, and reduce heat bloom. A user may review and/or modify a treatment plan, particularly, one generated by an algorithm, prior to application of the laser photocoagulation treatment.

The treatment plan is registered with the retina 210 in order to apply accurately the laser treatments to the selected locations. Thus, the selected treatment locations, such as selected locations 260, 270, and 280, are registered with the real-time image of the retina 210 such that, when the laser photocoagulation treatment is performed, the actual locations for which treatment is desired are struck by the laser beam. Registration may be made, for example, by an eye-tracking device with the use of retina features, such as blood vessels or the macula. Accurate positioning of the selected treatment locations may be made with reference to the locations and shapes of the retinal features. Various eye-tracking devices are known in the art.

Selected locations may be represented in different ways. For example, selected treatment locations 260 and 270 (unfilled circles) represented selected, but untreated locations, whereas selected treatment location 280 (filled circle) represented a selected and treated location. Although the example shows untreated locations as an unfilled circle and a treated location as a filled in circle, these indicators are provided merely as an example. The treated and untreated locations may be indicated in any desired way. For example, symbols having any desired shape, colors, text, or any other type of indication may be used to differentiate treated from untreated locations.

FIG. 2 also shows a laser target indication 290. In some instances, the laser target indication is light reflected off of the retina 210. The reflected light forming the laser target indication 290 may be transmitted from the laser delivery device 120. For example, the light transmitted by the laser delivery device 120 to define the laser target indication 290 may be a low energy light that identifies where a laser from the laser delivery device 120 would strike the retina 210 if the laser were fired. In some implementations, the laser target indication 290 may be utilized by a user to determine a location where the laser would impinge upon the retina 210. In some implementations, the system 100 may automatically recognize the laser target indication 290, such as by graphical recognition techniques or other image processing techniques. For example, the processor 160 may utilize software that can identify the target on the displayed image of the retina 210.

In other implementations, a position on the retina 210 of the laser target indication 290 may be determined by three dimensional data of the position of the laser delivery device 120 relative to the eye. The position on the retina 210 of the laser target indication 290 may be determined, for example, based on tracking of a longitudinal axis and distal end location and/or axial orientation of laser delivery device 120 relative to the position of the retina 210.

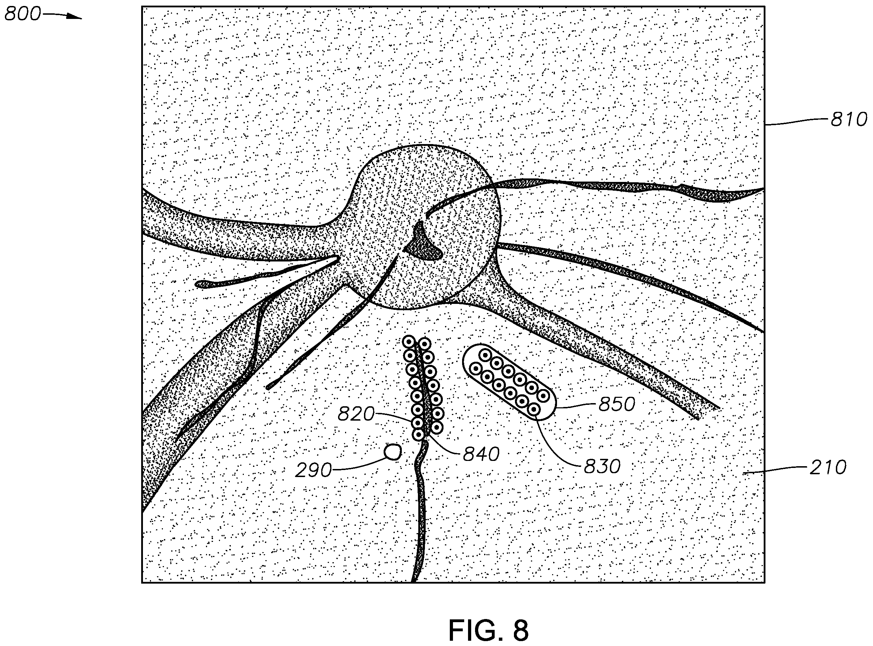

With the use of the OCT data taken along one or more lines (such as line 240) (or other types of data discussed herein or otherwise within the scope of the disclosure), a map or matrix of selected treatment locations forming part of a treatment plan is produced. FIG. 8 shows an example image 800. The image 800 includes a primary view 810 that may be similar to the primary view 220 shown in FIG. 2. Although not shown, the image 800 may also include a detail view similar to the detail view 230. In this example, FIG. 8 shows the same portion of the retina 210 shown in FIG. 2.

The image 800 also includes a plurality of selected treatment locations 820 and 830 for laser photocoagulation treatment. Each of the selected treatment locations 820 and 830 may be selected as explained above with respect FIG. 2. Further, in the example shown, the selected treatment locations 820 and 830 are untreated locations. This is observable based on the indication for each of the selected treatment locations 820 and 830. The collection of selected treatment locations 820 and 830, as well as any other desired information, forms the heads-up display. The heads-up display forms an overlay of data onto the real-time retinal image. Further, in some implementations, at least some of the information included in the heads-up display is registered with the retina 210, such as in the case of selected treatment locations 820 and 830. The heads-up display provides a convenient disclosure of information to a user that includes both the real-time image of a surgical site as well as information associated with the defined treatment plan. Further, display of information in this manner avoids the necessity of a user, such as a surgeon, having to look at different informational displays in order to understand the treatment and keep track of the surgical site. Inclusion of this information in a single location permits a user to maintain his or her attention on the task at-hand as well as reduce the time of a surgical procedure. Inclusion of this information also provides the user with a means of recording the areas that have been treated as well as those that remain untreated and enabling the user to keep track of where they are in their treatment plan. This feature is useful when, for example, subthreshold photocoagulation settings are used, as the treated tissues do not show a visually-apparent indication upon application of subthreshold photocoagulation.

The collection and storage of the information related to the selected treatment locations allows the surgeon to avoid having to remember the details associated with the treatment plan, as the treatment plan remains stored. This is beneficial, for example, if an unexpected or emergency event arises during a surgical procedure. The surgeon can address the unexpected or emergency event and, thereafter, proceed to executing the treatment plan at the point where the surgeon deviated to address the unexpected or emergency event. Without being able to track the treatment plan real time during the surgical procedure, which may include tracking of what treatment locations have already been treated and those remaining to be treated, subthreshold laser treatment would be difficult if not impossible to accomplish, as subthreshold treatments are not visible to the naked eye.

The selected treatment locations 820 may be located to treat abnormality 840, whereas selected treatment locations 830 may be located to treat another abnormality 850. Laser target indication 290 is also present in the image 800. Again, the laser target indication 290 identifies a location where laser energy will strike the retina 210 if a treatment laser were fired.

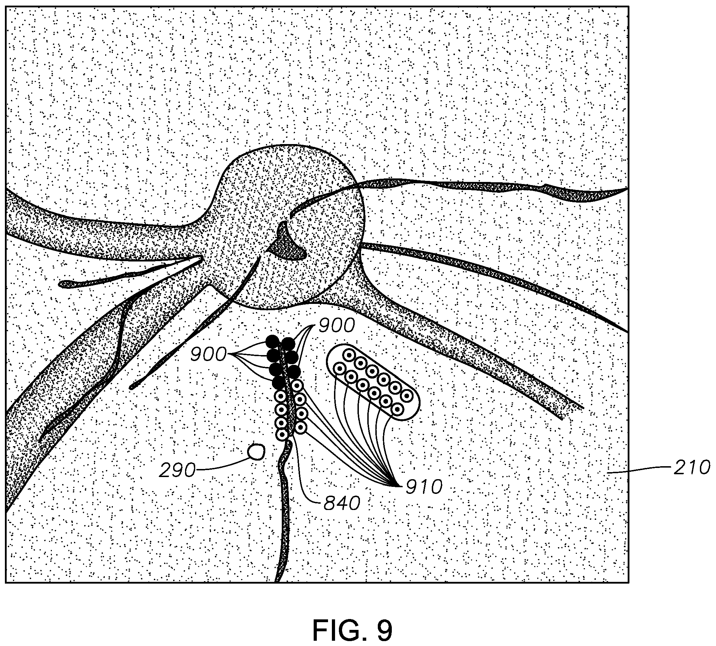

In some implementations, execution of a treatment plan may be entirely automated. For example, in some instances, positioning of the laser delivery device 120 may be controlled by the laser control device 110. The laser control device 110 may move the laser delivery device 120 to apply laser energy to each of the selected treatment locations, such as selected treatment locations 820 and 830 shown in FIGs. 8 and 9. Utilizing eye tracking coupled with the pre-programmed treatment locations , the laser control device 110 directs the laser delivery device 120 to each of the selected treatment locations 820 and 830 such that the laser target indication 290 overlays one of the selected treatment locations. In some instances, eye tracking and image processing determines a location of where laser radiation would strike the retina 210 upon firing the laser based on observation of the laser target indication 290.

Once a selected treatment location is accurately targeted (such as by registration of the laser target indication 290 with the selected treatment location), the laser control device 110 would fire a laser according to the determined laser parameters (also forming part of the treatment plan) determined for each of the selected treatment locations. Once laser photocoagulation treatment has been applied to one selected treatment location, the laser control device 110 may systematically direct the laser delivery device 120 to target and treat another selected treatment location. Further, upon completion of treatment of a selected treatment location, the system 100 updates the treatment plan. As part of the update to the treatment plan, the system 100 may alter the visual indicator of the selected treatment location to indicate treatment has been made. In some instances, when a selected treatment location has been treated, the treatment plan may be updated such that subsequent treatment of the same selected treatment location is prohibited.

As shown in the examples of FIGs. 8 and 9, the visual indicator reflecting treatment has occurred is a filled in circle. For example, FIG. 9 shows several selected treatment locations 900 filled in, indicating treatment has occurred, and several other selected treatment locations 910 unfilled, indicating that treatment has not yet occurred. However, any visual indicator may be used for this purpose. Particularly, any visual indicator may be used that is distinguishable from a visual indicator of a selected treatment location that has not yet undergone treatment. Once all of the selected treatment locations have been treated, the system 100 may provide an indication to the user. For example, the system 100 may provide an audible indication, a visual indication, or both.

In other implementations, execution of treatment plan may be a user-guided semi-automated process. Particularly, a user, such as a surgeon, may manipulate the laser delivery device 120 to align with the one or more selected treatment locations. Once a selected treatment location and the laser delivery device 120 are properly aligned, the system 100 automatically fires treatment laser 155 with the predetermined laser parameters according to the treatment plan. Alignment of a selected treatment location and the laser delivery device 120 may be determined using image processing and eye tracking. For example, tracking a position of the laser target indication 290 may be utilized to determine when the laser delivery device 120 is aligned with a selected treatment location. Once treated, the treatment plan is updated. This update may include changing or altering the indicator for the selected treatment location to indicate treatment has occurred. Updating may also include prohibiting further treatment of the selected treatment location, even if the laser delivery device 120 again becomes aligned with the location. This type of safeguard prevents a treatment location from being treated more than once. Such a treatment regime may be referred to as a user guided semi-automated regime. This process may be continued until all selected treatment locations are treated.

A further manner of treating the selected treatment locations according to the treatment plan may be entirely manual. That is, in some implementations, a user manually aligns the laser delivery device 120 with a selected treatment location, such as by aligning the laser target indication 290 with a selected treatment location. In some instances, the system 100 may indicate when the laser delivery device 120 is aligned with a selected treatment location. The user would then fire the treatment laser 155. Once fired, the system 100, such as with the laser control device 110, would control the firing of the laser so as to conform to the parameters of the treatment plan. When treatment of a selected treatment location is complete, the treatment plan is updated. For example, the system 100 would note that the particular selected treatment location has been treated, preventing further treatment of the location, and changing the indication of the selected treatment location to indicate that treatment has occurred.

Utilizing the system 100 to provide the laser treatment in any of the ways described herein is important due to the difficulty a user may have in visually identifying where a laser treatment has been applied. This may be because, for example, a location that has been treated with the appropriate amount of laser energy may not be discernable from a non-treated location. Thus, one the laser is fired, the system 100 (such as the laser control device 110 thereof) controls application of the laser radiation according to the treatment plan in order to provide an improved treatment as well as records which locations have and have not been treated. This improves safety and efficacy of the surgical procedure.

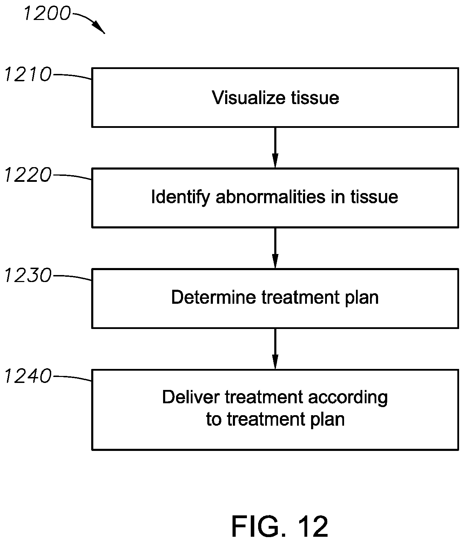

FIG. 12 shows an example method 1200 for treating a tissue. Although method 1200 is described with reference to treatment of an ocular tissue, as explained above, the method 1200 is applicable to treatment of any tissue both within and outside of ophthalmology. That is, the method 1200 may be used in other medical fields other than ophthalmology to treat other types of maladies. Further, as explained above, the scope of the disclosure, including method 1200, is not limited to laser photocoagulation. Rather, the use of the principles described herein may be used with other types of treatments. Thus, these other uses are also within the scope of the disclosure.

At 1210, a tissue is visualized. In the present example, an ocular tissue is visualized. Visualization may be performed with numerous techniques operable to identify abnormalities with the visualized tissue. For example, the visualization may be performed using OCT, infrared imaging, and retinal tomography. Other types of visualizations may also be used in order to identify tissue abnormalities.

In some implementations, visualization may be accomplished using a device external to the eye. Visualization includes obtaining imaging data of a tissue that may be used to determine the existence of any tissue abnormalities. For example, in the case of OCT, imaging data in the form of OCT data may be obtained with an OCT device that is external to the eye. Particularly, OCT data may be obtained from a microscope, such as microscope 140 described above, having OCT capability. Thus, in some instances, the OCT data obtained from visualization of the ocular tissue through the cornea and lens of a patient's eye. In some instances, the visualization data may be obtained by a device or probe at least partially inserted into the eye. An imaging device, such as imaging device 130, may be used to obtain this type of visualization data. For example, again in the case of OCT, an OCT probe may be inserted at least partially into the eye in order to obtain OCT data of the ocular tissue in question, such as, for example, the retina.

Further, in some implementations, the visualization information may be accomplished manually. For example, in some instances, at least some of the imaging data may be obtained by manual operation of an imaging device (e.g., an OCT probe) by a user. The user may guide the imaging device and obtain imaging data at one or more desired locations. In other instances, the imaging data may be obtained automatically according to a predetermined algorithm. For example, an imaging device may obtain imaging data by executing a predetermined algorithm that obtains imaging data from a preselected area of the tissue. In the instance of OCT, the imaging data may be automatically obtained along a plurality of scan lines to sufficiently cover any desired area of the tissue. The imaging data, whether obtained manually or automatically, may then be stored. The stored data may subsequently be analyzed for the existence of any abnormalities. In some instances, the imaging data may be stored digitally.

At 1220, the imaging data is used to identify potential abnormalities of the tissue. For example a system, such as system 100, may be used to identify potential abnormalities. More particularly, in some instances, a control device that may be similar to laser control device 110, operating with one or more algorithms that may be contained in an application, such as an application similar to laser control application 180, may be used to identify potential abnormalities. In some implementations, one or more algorithms may be used to analyze the obtained imaging data and determine whether one or more abnormalities exist. Thus, in some implementations, identification of abnormalities may be performed electronically without selection input from a user.

Image processing algorithms, such as one or more of the algorithms explained above, may be used to determine the presence of different types of abnormalities. As explained above, example metrics that may be used to determine the presence of one or more abnormalities includes a thickness, an intensity, an intensity gradient, a phase, a speckle size, a vascular density, a blood flow velocity, an oxygenation, an elasticity, a birefringence property, a size, a volume, a concavity/convexity, and/or a radius of curvature of one or more retinal layers. For example, in the case of retinal laser photocoagulation, image processing algorithms may be used to determine the one or more areas of the retina that is/are candidates for treatment. Candidate areas of the tissue may be identified by the detection of various abnormalities that may be present, such as, for example, venous occlusions, macular edema, microvascular abnormalities, retinal breaks and tears, and ocular tumor to name a few. The location of the abnormalities may be identified and stored, such as in a memory device similar to memory device 170 of example system 100.

The locations of the identified abnormalities may be determined using an eye-tracking device and/or algorithm. Eye tracking permits the registration of the image data and the precise location of the tissue from which the data were obtained. Thus, during the treatment portion of a surgical procedure, the locations of the identified abnormalities may be known in order to facilitate accurate aiming and treatment application to those locations. For example, in some instances, the locations of the abnormalities may be accurately overlaid onto a real-time image of the tissue to permit accurate application of a treatment therapy, such as a laser photocoagulation treatment.

Registration of selected treatment locations of one or more abnormalities may be accomplished with the use of identifying characteristics of a tissue or physiological characteristic. For example, in the context of a retina, features of the retina, such as retinal vessels, may be used to accurately register imaging data to a real-time image of a retina.

At 1230, a treatment plan to accomplish an intended treatment is determined. Determination of a treatment plan may include identifying locations of a tissue for treatment, i.e., selected treatment locations, identifying treatment parameters for a treatment to be applied to the selected treatment locations, and an order of performing the treatment. A treatment plan may also include other aspects, such as, for example, an order in which the selected treatment locations are treated. A processor running an application, such as processor 160 and laser control application 180 of the example system 100, may be used to determine a treatment plan based on obtained imaging data.

In the example of retinal laser photocoagulation, the treatment plan may include determination of laser parameters that control the amount and manner in which laser energy is applied to a particular selected treatment location. For example, a treatment plan for laser photocoagulation may include parameters such as, for example, laser power, a number of locations to be treated, positions of the selected treatment locations, a size of the selected treatment locations, areas where treatment is to be avoided ("exclusion zones"), and/or a time period laser energy is applied to a location. Other parameters may also be determined.

In some implementations, a treatment plan may be determined according to an algorithm. In some instances, the treatment plan may be determined exclusively by an algorithm. The algorithm may determine a treatment plan based on, for example, the type of abnormality, a size of the abnormality, a location of the abnormality. Other characteristics may also be used to determine the treatment plan. For treatments involving laser energy, an algorithm utilized to develop a treatment plan may optimize laser parameters in order to improve procedure effectiveness, optimize an amount of time to perform the procedure, control and/or reduce cellular necrosis, eliminate or minimize vision loss, and reduce or eliminate heat bloom.

The treatment plan may form part of a heads-up display that may be overlaid onto a real-time image of the tissue being treated. The treatment plan, too, may include registration data that provides for accurately applying the parameters of the treatment plan onto a real-time image. For example, the registration provides for accurately locating on a real-time image the selected treatment locations. As a result, accurate treatment is applied to the tissue.

At 1240, treatment is delivered according to the treatment plan. In some implementations, application of the treatment plan may be fully automated. For example, the treatment plan may be delivered exclusively by a treatment device with little to no input from a user. For example, a device may include eye-tracking capabilities. The treatment device utilizes a treatment plan containing registration information and overlays the treatment plan onto a real-time image of the tissue being treated. The treatment device controls a position of a treatment instrument, such as, for example, a laser delivery device. The treatment device applies treatment according to the treatment plan. For example, the treatment device applies a treatment according to determined parameters for each selected treatment site and according to an order established by the treatment plan. The treatment plan may be updated as the procedure progresses, such as by tracking which selected treatment locations have been treated and which remain untreated. At the conclusion of the treatment, a user may be notified, such as with an audible and/or visual notification.

In other implementations, delivery of treatment according to a treatment plan may be partially automated. In some instances, a user may manually aim a treatment delivery device or instrument (e.g., a laser delivery device) and the instrument may be made to apply treatment according to the treatment plan by a treatment control device. For example, the treatment control device may be operable to detect when the treatment delivery device is aligned with a particular selected treatment location and automatically apply a treatment thereto according to the treatment plan. Thus, while a user may manually maneuver a treatment delivery device, actual execution of the treatment plan is accomplished by a treatment control device. Image processing may be utilized to monitor a target location of a treatment delivery device relative to selected treatment locations. When alignment between the target location and a selected treatment location occurs, application of treatment may be performed automatically.

According to still other implementations, application of a treatment plan may be accomplished substantially manually. For example, a user may guide a treatment delivery device and, when a treatment delivery device is aligned with a selected treatment location, a notification may be provided to the user. The user may then trigger application of the treatment to the selected treatment location. However, the actual application of the treatment is controlled by a treatment control device.

Although FIG. 12 illustrates one implementation of a method for treating a tissue, other methods for treating a tissue may include fewer, additional, and/or a different arrangement of operations. For example, another example method for treating a tissue may additionally call for presenting one or more selected treatment locations to a user for review or modification. Another example method may include presenting a treatment plan to a user for review or modification. In still other instances, another example method may include displaying a real-time image of a tissue for treatment with a registered representation of a treatment plan. The displayed information may be updated as the treatment progresses. For example, upon treatment of a selected treatment location, a representation of the selected treatment location may be updated both visually and within the treatment plan to indicate that treatment of that selected treatment location has occurred.

Although the disclosure provides numerous examples, the scope of the present disclosure is not so limited. Rather, a wide range of modification, change, and substitution is contemplated in the foregoing disclosure. It is understood that such variations may be made to the foregoing without departing from the scope of the present disclosure.

## Claims

1. A surgical optimization system (100) comprising:
an imaging device (130) adapted to receive imaging data of a portion of retinal tissue at an anticipated treatment location for a patient; and
a laser treatment delivery device (120) adapted to apply a treatment to the retinal tissue at the treatment location; and
a treatment control device (110), the imaging device and the treatment device coupled to the treatment control device, the treatment control device comprising:
a processor (160) adapted to:
determine for the portion of the retinal tissue an expected normal range of thicknesses of the neurosensory retina based on data from a database containing thickness measurements of the neurosensory retina of patients with similar characteristics, and using the normal range of thicknesses to generate a threshold value range based on a percentage difference in the neurosensory retina thickness compared to adjacent areas;
identify the presence of one or more abnormalities of the retinal tissue based on the received imaging data when an area of the patient's neurosensory retina has a thickness greater than or less than the threshold value, the one or more abnormalities being identifiable as an area along the neurosensory retina with a thickness different from adjacent areas that is greater than or less than the threshold value range; and
identify from the imaging data a vascular density associated with the one or more abnormalities of the retina tissue based on a detected vascular density value;
determine a type of abnormality of the one or more abnormalities of the retina tissue based on the vascular density value, wherein vascular density of a particular value is representative of a particular type of abnormality;
determine one or more laser treatment parameters based on the type of abnormality;
deliver a treatment to the one or more abnormalities according to a treatment plan using the one or more laser treatment parameters via the treatment delivery device.

2. The system of claim 1, wherein the treatment control device (110) further comprises a user interface (190), the user interface adapted to receive the treatment plan.

3. The system of claim 1, wherein the processor (160) of the treatment control device (110) is further adapted to determine the treatment plan using the identified one or more abnormalities.

4. The system of claim 3, wherein the processor (160) is further adapted to update the treatment plan during the course of the treatment.

5. The system of claim 1, wherein a type of identified abnormality comprises one of a venous occlusion, a macular edema, a microvascular abnormality, a retinal break, a retinal tear, or an ocular tumor.

6. The system of claim 1, wherein the imaging device (130) is an optical coherence tomography, OCT, device.

7. The system of claim 1, wherein the treatment parameters include one of a location to apply a treatment, a size of the location to be treated, locations excluded from treatment, and a laser power to be used for treatment.

8. The system of claim 1, wherein delivery of the treatment to the one or more abnormalities according to the treatment plan via the treatment delivery device is performed autonomously by the treatment control device.

9. The system of claim 1, wherein delivery of the treatment to the one or more abnormalities according to the treatment plan via the treatment delivery device is performed upon receipt of a user input.

10. The system of claim 9, wherein the user input comprises alignment of a target indicator with a treatment location of the abnormality.

## Patentansprüche

1. Chirurgisches Optimierungssystem (100), umfassend:
eine Bildgebungsvorrichtung (130), die dafür ausgelegt ist, Bildgebungsdaten eines Teils des Netzhautgewebes an einer voraussichtlichen Behandlungsstelle für einen Patienten zu empfangen; und
eine Laserbehandlungs-Durchführungsvorrichtung (120), die dafür ausgelegt ist, eine Behandlung auf das Netzhautgewebe an der Behandlungsstelle anzuwenden; und eine Behandlungssteuervorrichtung (110), wobei die Bildgebungsvorrichtung und die Behandlungsvorrichtung mit der Behandlungssteuervorrichtung gekoppelt sind, wobei die Behandlungssteuervorrichtung umfasst:
einen Prozessor (160), der ausgelegt ist zum:
Bestimmen, für den Teil des Netzhautgewebes, eines erwarteten normalen Dickenbereichs der neurosensorischen Netzhaut basierend auf Daten aus einer Datenbank, die Dickenmessungen der neurosensorischen Netzhaut von Patienten mit ähnlichen Merkmalen enthält, und Verwenden des normalen Dickenbereichs, um einen Schwellenwertbereich zu erzeugen, basierend auf einem prozentualen Unterschied in der Dicke der neurosensorischen Netzhaut im Vergleich zu benachbarten Bereichen;
Identifizieren des Vorhandenseins einer oder mehrerer Anomalien des Netzhautgewebes basierend auf den empfangenen Bilddaten, wenn ein Bereich der neurosensorischen Netzhaut des Patienten eine Dicke aufweist, die größer oder kleiner als der Schwellenwert ist, wobei die ein oder mehreren Anomalien als ein Bereich entlang der neurosensorischen Netzhaut mit einer Dicke identifizierbar sind, die sich von benachbarten Bereichen unterscheidet und größer oder kleiner als der Schwellenwertbereich ist; und
Identifizieren, anhand der Bildgebungsdaten, einer Gefäßdichte, die mit den ein oder mehreren Anomalien des Netzhautgewebes verknüpft ist, basierend auf einem erkannten Gefäßdichtewert;
Bestimmen eines Typs der Anomalie der ein oder mehreren Anomalien des Netzhautgewebes basierend auf dem Gefäßdichtewert, wobei eine Gefäßdichte mit einem bestimmten Wert für einen bestimmten Typ von Anomalie repräsentativ ist;
Bestimmen eines oder mehrerer LaserBehandlungsparameter basierend auf dem Typ der Anomalie;
Durchführen einer Behandlung der ein oder mehreren Anomalien gemäß einem Behandlungsplan unter Verwendung der ein oder mehreren Laserbehandlungsparameter über die Behandlungsdurchführungsvorrichtung.

2. System nach Anspruch 1, wobei die Behandlungssteuervorrichtung (110) ferner eine Benutzerschnittstelle (190) umfasst, wobei die Benutzerschnittstelle dafür ausgelegt ist, den Behandlungsplan zu empfangen.

3. System nach Anspruch 1, wobei der Prozessor (160) der Behandlungssteuervorrichtung (110) ferner dafür ausgelegt ist, den Behandlungsplan anhand der identifizierten ein oder mehreren Anomalien zu bestimmen.

4. System nach Anspruch 3, wobei der Prozessor (160) ferner dafür ausgelegt ist, den Behandlungsplan während des Verlaufs der Behandlung zu aktualisieren.

5. System nach Anspruch 1, wobei ein Typ der identifizierten Anomalie einen Venenverschluss, ein Makulaödem, eine mikrovaskuläre Anomalie, einen Netzhautbruch, einen Netzhautriss oder einen Augentumor umfasst.

6. System nach Anspruch 1, wobei die Bildgebungsvorrichtung (130) eine optische Kohärenztomographie, OCT (Optical Coherence Tomography), -Vorrichtung ist.

7. System nach Anspruch 1, wobei die Behandlungsparameter eines von der zu behandelnden Stelle, der Größe der zu behandelnden Stelle, von der Behandlung ausgeschlossene Stellen bzw. der für die Behandlung zu verwendenden Laserleistung beinhalten.

8. System nach Anspruch 1, wobei die Durchführung der Behandlung der ein oder mehreren Anomalien gemäß dem Behandlungsplan über die Behandlungsdurchführungsvorrichtung autonom durch die Behandlungssteuervorrichtung erfolgt.

9. System nach Anspruch 1, wobei die Durchführung der Behandlung der ein oder mehreren Anomalien gemäß dem Behandlungsplan über die Behandlungsdurchführungsvorrichtung nach Empfang einer Benutzereingabe durchgeführt wird.

10. System nach Anspruch 9, wobei die Benutzereingabe die Ausrichtung eines Zielindikators auf eine Behandlungsstelle der Anomalie umfasst.

## Revendications

1. Système d'optimisation chirurgicale (100) comprenant :
un dispositif d'imagerie (130) adapté pour recevoir des données d'imagerie d'une partie du tissu rétinien à un emplacement de traitement anticipé pour un patient ; et
un dispositif d'administration de traitement au laser (120) adapté pour appliquer un traitement au tissu rétinien à l'emplacement du traitement ; et
un dispositif de commande du traitement (110), le dispositif d'imagerie et le dispositif de traitement étant couplés au dispositif de commande du traitement, le dispositif de commande du traitement comprenant :
un processeur (160) adapté pour :
déterminer pour la partie du tissu rétinien une plage normale attendue d'épaisseurs de la rétine neurosensorielle sur la base de données contenant des mesures d'épaisseur de la rétine neurosensorielle de patients présentant des caractéristiques similaires, et utiliser la plage normale d'épaisseurs pour générer une plage de valeurs seuils basée sur une différence en pourcentage de l'épaisseur de la rétine neurosensorielle par rapport à des zones adjacentes ;
identifier la présence d'une ou plusieurs anomalies du tissu rétinien sur la base des données d'imagerie reçues lorsqu'une zone de la rétine neurosensorielle du patient a une épaisseur supérieure ou inférieure à la valeur seuil, la ou les anomalies étant identifiables comme une zone le long de la rétine neurosensorielle avec une épaisseur différente des zones adjacentes qui est supérieure ou inférieure à la plage de valeurs seuil ; et
identifier, à partir des données d'imagerie, une densité vasculaire associée à l'anomalie ou aux anomalies du tissu rétinien, sur la base d'une valeur de densité vasculaire détectée ;
déterminer un type d'anomalie de la ou des anomalies du tissu rétinien sur la base de la valeur de densité vasculaire, la densité vasculaire d'une valeur particulière étant représentative d'un type particulier d'anomalie ;
déterminer un ou plusieurs paramètres de traitement au laser sur la base du type d'anomalie ;
administrer un traitement à l'anomalie ou aux anomalies conformément à un plan de traitement utilisant le ou les paramètres de traitement au laser par l'intermédiaire du dispositif d'administration de traitement.

2. Système selon la revendication 1, le dispositif de commande du traitement (110) comprenant en outre une interface utilisateur (190), l'interface utilisateur étant adaptée pour recevoir le plan de traitement.

3. Système selon la revendication 1, le processeur (160) du dispositif de commande du traitement (110) étant en outre adapté pour déterminer le plan de traitement à l'aide de la ou des anomalies identifiées.

4. Système selon la revendication 3, le processeur (160) étant en outre adapté pour mettre à jour le plan de traitement au cours du traitement.

5. Système selon la revendication 1, un type d'anomalie identifiée comprenant une occlusion veineuse, un œdème maculaire, une anomalie microvasculaire, une rupture de la rétine, une déchirure de la rétine ou une tumeur oculaire.

6. Système selon la revendication 1, le dispositif d'imagerie (130) étant un dispositif de tomographie par cohérence optique, OCT.

7. Système selon la revendication 1, les paramètres de traitement comprenant un paramètre parmi un emplacement à traiter, une taille de l'emplacement à traiter, des emplacements exclus du traitement et une puissance du laser à utiliser pour le traitement.

8. Système selon la revendication 1, l'administration du traitement à l'anomalie ou aux anomalies conformément au plan de traitement par l'intermédiaire du dispositif d'administration de traitement étant effectuée de manière autonome par le dispositif de commande du traitement.

9. Système selon la revendication 1, l'administration du traitement à l'anomalie ou aux anomalies conformément au plan de traitement par l'intermédiaire du dispositif d'administration de traitement étant effectuée à la réception d'une entrée de l'utilisateur.

10. Système selon la revendication 9, l'entrée de l'utilisateur comprenant l'alignement d'un indicateur de cible avec un emplacement de traitement de l'anomalie.
